# EUROPÄISCHE PATENTSCHRIFT

(11) **EP 0 975 268 B1**
(45) Veröffentlichungstag und Bekanntmachung des Hinweises auf die Patenterteilung: **04.06.2003**
(21) Anmeldenummer: 98922675.8
(22) Anmeldetag: 09.04.1998
(51) Int. Cl.: A61B 17/22

(54) **METALLSONDE ZUR VERWENDUNG BEI DER INTRAKORPORALEN LITHOTRIPSIE**
METAL PROBE FOR INTRACORPOREAL LITHOTRIPSY
SONDE METALLIQUE A UTILISER EN LITHOTRIPSIE INTRACORPORELLE

(30) Priorität: 15.04.1997 DE 19715698
(43) Veröffentlichungstag der Anmeldung: 02.02.2000
(73) Patentinhaber: Engel, Konrad Dr.med., 83674 Gaissach (DE)
(72) Erfinder: Engel, Konrad Dr.med., 83674 Gaissach (DE)
(74) Vertreter: Haft, von Puttkamer, Berngruber, Czybulka
(86) Internationale Anmeldenummer: EP9802077
(87) Internationale Veröffentlichungsnummer: WO98046146

(56) Entgegenhaltungen:
- EP-A- 0 331 313
- WO-A-93/16646
- DE-A- 2 032 501
- DE-A- 3 707 567
- DE-U- 7 705 947
- FR-A- 2 317 903

## Beschreibung

Die Erfindung bzieht sich auf eine Metallsonde zur Verwendung bei der intrakorporalen Lithotripsie gemäß dem oberbegriff des Patentanspruchs 1.

Die Metallsonde ist hierbei in das Lumen eines Endoskops einführbar; die ein distales Ende der Metallsonde bildende Sondenspitze wird für eine Zertrümmerung von Körpersteinen durch eine pulsierende Translationsbewegung der Sondenspitze genutzt.

Metallsonden der vorgenannten Art werden beispielsweise bei einem Lithotripter einer Ausbildung gemäß der EP 0 317 507 B1 verwendet. Bei diesen Lithotriptern wird die an die Sonde zur Übertragung kommende Stoßenergie aus dem Schlagimpuls eines pneumatisch angetriebenen Schlagteils gegen einen Sondenkopf vergrößerten Querschnitts an dem proximalen Ende der Sonde erhalten, so dass mit der daraus resultierenden, die Sonde durchlaufenden Stoßwelle eine Translationsbewegung der Sondenspitze erhalten wird. Diese Stoßwelle kann für eine mittels des verwendeten Endoskops intrakorporal durchgeführte Zertrümmerung von Körpersteinen, wie Nieren-, Harnleiter- oder Blasensteinen, benutzt werden.

Die bisher bspw. für eine Zertrümmerung von Harnleitersteinen verwendeten Sonden besitzen eine relativ stumpfe Spitze, mit welcher sie weitgehend senkrecht auf den anfangs durch die Harnleiterwand fixierten Stein auftreffen. Dabei kommt es häufig vor, daß der Stein während der Einwirkung der Stoßwellen aus seiner Fixierung gelöst und in Richtung der Niere katapultiert wird, da der Harnleiter oberhalb des Steines durch den aus der Niere nachfließenden Harn meistens deutlich erweitert ist. Eine solche für die Steinzertrümmerung unerwünschte Steinpropulsion wird daher häufig durch ein ergänzendes Fixieren des Steines zu verhindern versucht, nämlich durch ein sogenanntes "Basketing", bei welchem in den Harnleiter ein korbähnlicher Steinfänger,ein sog. "Dormiakörbchen", zusätzlich vorgeschoben wird.

Diese für das Arbeiten mit solchen Sonden somit nachteilige und allenfalls mit solchen aufwendigen Hilfsmaßnahmen gegensteuerbare Steinpropulsion erfährt eine weitere Beeinträchtigung im Umfang der bei der Zertrümmerung von Harnleitersteinen zur Verfügung stehenden Ureteroskope. Bei diesen Ureteroskopen wird etwa die Hälfte des an dem distalen Ende maximal bis zu 4 mm (12 Charriere) weiten Querschnitts durch einen die Sonde aufnehmenden Arbeitskanal beansprucht, während die andere Hälfte des Querschnitts eine starre, stabförmige Optik oder auch halbstarre Fieberglasfasern und einzelne Lichtfaserbündel aufnimmt. Diese seitlich und damit nicht im Zentrum des Querschnitts angeordnete Optik besitzt keinen wesentlichen Weitwinkeleffekt, so daß die Spitze der in das Ureteroskop vorgeschobenen Sonde nur am Rand des Gesichtsfeldes entlang der Harnleiterwand sichtbar wird. Zum Aufsetzen der Sondenspitze auf die Mitte der sichtbaren Steinoberfläche ist daher meistens ein leichtes Kippen des Ureteroskops erforderlich. Dadurch kommt es dann aber zu einer Änderung der Blickrichtung gegen die Harnleiterwand. Dieser Effekt wird durch eine stets vorhandene Innenwölbung der zwischen dem Harnleiterstein und dem distalen Ende des Ureteroskops nicht aufgedehnten Harnleiterwand verstärkt, sodaß die Sondenspitze und der Stein oft völlig aus dem Gesichtsfeld verschwinden und daher eine Steirizertrümmerung unter einer ausreichenden Sichtkontrolle oft unmöglich ist.

Aus der DE 2032501A ist eine Metallsonde zum Zertrümmern von Körpersteinen bekannt, von der die Erfindung ausgeht. Diese bekannte Metallsonde weist ein Rohr auf, in dessen Inneres ein Endoskop eingeführt werden kann, mit dem über eine seitliche Aussparung an der Sondenspitze eine Beobachtung des Operationsfeldes gestattet wird. Das Rohr der Sonde ist an der Sondenspitze konisch ausgebildet, wobei dieser Konus als Schlagspitze zur Zertrümmerung des Steines dient. Gleichzeitig können über diese Spitze auch die Trümmer des Steines abgesaugt werden.

Bei dieser bekannten Metallsonde ist es zudem möglich, in den Hohlraum des Rohres eine schnabelförmige Greifvorrichtung einzuführen, mit der der Stein manuell erfasst werden kann.

Aus der EP 0331313A1 ist eine Sonde bekannt, mit der Fettgewebe im Rahmen einer kosmetischen Operation entfernt werden kann. Die beschriebene Sonde ist als Hohlrohr ausgebildet, die an ihrer Spitze mehrere Öffnungen aufweist, mit denen das Fettgewebe abgeschabt und durch den Hohlraum der Sonde abgesaugt werden kann.

Ausgehend von der DE2032501A liegt der Erfindung die Aufgabe zugrunde, eine für die intrakorporale Lithotripsie geeignete Metallsonde bereitzustellen, mit der bei der Zertrümmerung insbesondere von Harnleitersteinen eine Propulsion der Steine relativ zu dem angrenzenden Harnleiter vermieden und der Stein bei der Zertrümmerung besser fixiert werden kann. Außerdem soll die Sicht auf den Stein bei der Fixierung und Zertrümmerung erhalten bleiben, wenn die Lithotripsie mittels eines herkömmlichen Ureteroskops durchgeführt wird.

Diese Aufgabe wird gemäß der vorliegenden Erfindung durch die Merkmale des Patentanspruches 1 gelöst.

Demgemäß ist die Sonde ein Vollkörper mit kostantem kreisrunden Querschnitt und mit einer abgerundeten Spitze, wobei die Sondenspitze nahe dem Sondenende mit wenigstens einer seitlichen Einkerbung versehen ist, die für ein seitwärts ausgerichtetes Fixieren des zu zertrümmernden Körpersteines gegen eine angrenzende Ductuswand und zu dessen Zertrümmerung ausgebildet ist.

Bei der erfindungsgemäßen Metallsonde wird somit durch die an der Sondenspitze seitlich ausgebildete Einkerbung oder vorteilhaft durch eine Mehrzahl solcher Einkerbungen entlang der Sondenspitze die Möglichkeit erhalten, einen an der Harnleiterwand anfänglich fixierten Harnleiterstein für die Dauer der Steinzertrümmerung durch die Ausübung eines leichten seitlichen Druckes mit der Sonde auf den Stein fortgesetzt fixiert zu halten, sodaß dadurch eine Propulsion des Steines während der Steinzertrümmerung vermieden wird. Durch die seitliche Anordnung der einen oder mehreren Einkerbungen bleibt dabei gleichzeitig das Vorschieben der Sonde durch das Lumen des Ureteroskops unbehindert und bleibt auch die Möglichkeit erhalten, mit einem im wesentlichen stumpf ausgeführten Sondenende eine wie bisher übliche Steinzertrümmerung vorzunehmen, bei welcher die Sondenspitze nicht seitlich an den Stein, sondern im wesentlichen senkrecht zu dem Stein geführt wird.

Weitere vorteilhafte und zweckmäßige Ausbildungen der erfindungsgemäßen Metallsonde ergeben sich aus den Unteransprüchen.

Ein Ausführungsbeispiel der Erfindung ist in der Zeichnung schematisch dargestellt und wird nachfolgend näher erläutert.
Es zeigen
- Fig. 1: eine Schemadarstellung der Sondenspitze,
- Fig. 2: eine Schnittansicht der Sondenspitze nach der Linie II-II in Fig. 1,
- Fig. 3: eine Schemadarstellung zur Erläuterung der Zertrümmerung eines Harnleitersteines unter Verwendung einer erfindungsgemäßen Sonde, die in das Lumen eines Ureteroskops eingesetzt ist,
- Fig. 4: eine Schemadarstellung zur Erläuterung der Zertrümmerung eines Harnleitersteines unter Verwendung einer Sonde gemäß dem Stand der Technik und
- Fig. 5: eine Schemadarstellung zur Erläuterung der Zertrümmerung eines Harnleitersteines unter Verwendung einer erfindungsgemäßen Sonde.

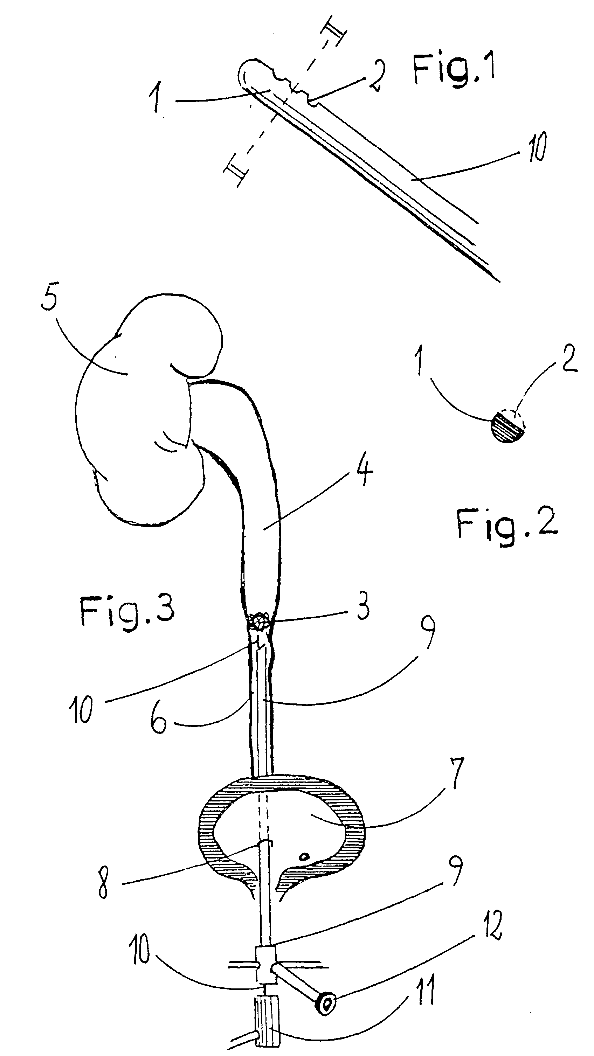

Gemäß der Darstellung in Fig. 1 ist eine zur Verwendung bei der intrakorporalen Stoßwellen-Lithotripsie vorgesehene Metallsonde 10 kreisrunden Querschnitts an ihrem distalen Ende mit einer Sondenspitze 1 ausgebildet, die seitliche Einkerbungen 2 aufweist. Die Einkerbungen 2 sind als Zylindersegmente ausgebildet, sodaß sich an jeder Einkerbung ein kreissegmentförmiger Querschnitt der Sonde gemäß der Darstellung in Fig. 2 ergibt. Die Tiefe jeder Einkerbung ist so bemessen, daß die Sondenspitze an dieser Stelle nicht brechen kann. Anstelle der Ausbildung als ein Zylindersegment kann jede Einkerbung auch als ein Quadersegment oder als ein Vieleck bzw. auch sägezahnförmig ausgebildet sein. Auch eine Kombination unter diesen verschiedenen Ausbildungen ist denkbar, sofern nicht nur wenigstens eine Einkerbung, sondern mehrere, dabei gleich ausgerichtete und entlang einer gemeinsamen Mantellinie der Sonde parallel zu deren Längsachse fluchtende Einkerbungen vorgesehen sind.

Zur Zertrümmerung bspw. eines Harnleitersteines 3, der gemäß der Darstellung in Fig. 3 in einem Harnleiter eingefangen ist, wird die Sonde 10 in das Lumen eines dabei verwendeten Ureteroskops 9 eingeführt. Die Sonde 10 ist durch das Handstück 11 eines Lithotripters bspw. einer Ausbildung gemäß der EP 0 317 507 gehalten. Bei diesen Lithotriptern wird durch ein pneumatisch angetriebenes Schlagteil eine auf die Sonde an ihrem proximalen Ende zur Übertragung kommende Stoßenergie erzeugt, welche in der Ausbildung einer die Sonde durchlaufenden Stoßwelle resultiert. Daneben kann die Sonde auch bei Lithotriptern verwendet sein, bei denen ein vergleichbares Schlagteil hydraulisch oder auch elektromagnetisch angetrieben wird.

Der Harnleiterstein 3 ist in einer Position fixiert, in welcher ein oberer Abschnitt 4 des Harnleiters als Folge der mit dem Stein erhaltenen Stauwirkung durch den aus der Niere 5 zulaufenden Harn deutlich erweitert ist. Ein unterer Abschnitt 6 des Harnleiters ist mit der Harnblase 7 verbunden, wobei in die betreffende Harnleitermündung 8 das distale Ende des Ureteroskops 9 soweit eingeführt ist, daß die über dieses distale Ende vorstehende Spitze der Sonde 10 nahe dem zu zertrümmernden Harnleiterstein 3 positioniert ist. Diese Positionierung der Sondenspitze kann über ein Okkular 12 des Ureteroskops 9 eingesehen werden.

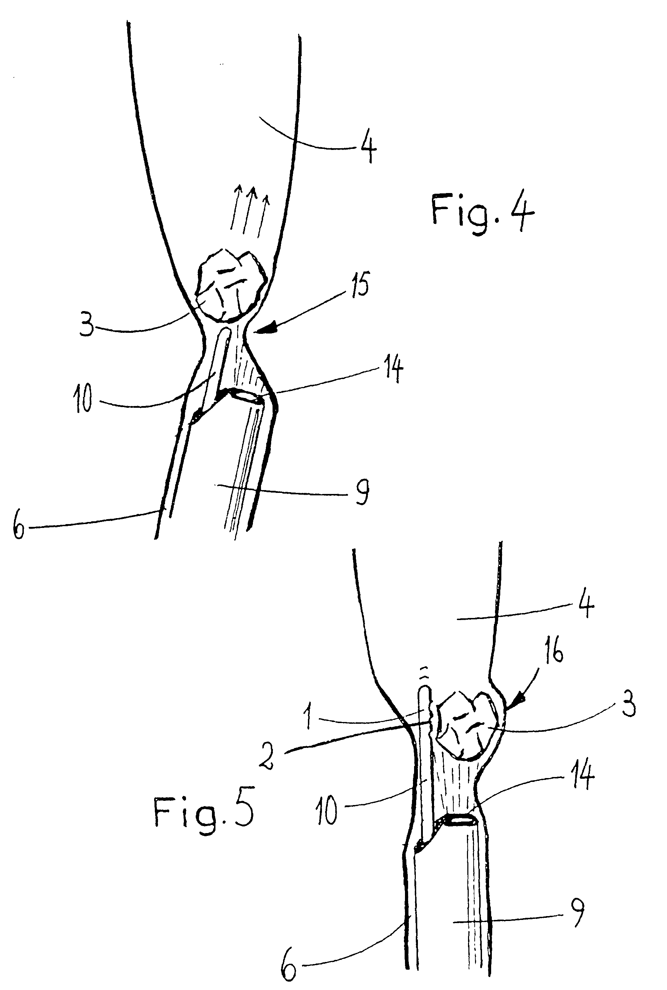

In der Schemadarstellung der Fig. 4 ist gezeigt, welche Verhältnisse bei der Zertrümmerung des Harnleitersteines 3 vorliegen können, sofern dabei eine Sonde herkömmlicher Ausführung mit einer relativ stumpfen Spitze verwendet wird. Beim Vorschieben des distalen Ende des Ureteroskops 9 erfährt der untere Harnleiterabschnitt 6 eine Aufdehnung (Bougierung), sodaß sich als deren Folge nahe dem Harnleiterstein 3 eine zirkuläre Innenwölbung 15 ergibt. Durch diese Innenwölbung ergibt sich eine Beeinträchtigung für das Sichtfeld der Optik 14 des Ureteroskops 9, die noch zusätzlich dadurch verstärkt werden kann, wenn für eine optimale Steinzertrümmerung versucht wird, die Sondenspitze auf die Mitte des Steines 3 auszurichten und dafür dann das distale Ende des Ureteroskops 9 aus der Längsachse des Harnleiterabschnitts 6 heraus gekippt wird. Durch diese Kippbewegung kann dabei gleichzeitig die Fixierung des Steines gelockert werden, sodaß der Stein eine Verschiebung in den erweiterten Harnleiterabschnitt 4 erfährt und es dabei zu einer Steinpropulsion hinein in diesen oberen Abschnitt des Harnleiters kommt, sobald mit der eigentlichen Steinzertrümmerung begonnen wird und es dabei zu einer Transiationsbewegung der Sondenspitze als Folge der die Sonde durchlaufenden Stoßwellen kommt.

Die Schemadarstellung der Fig. 5 veranschaulicht die vergleichbaren Verhältnisse, die bei der Zertrümmerung des Harnleitersteines 3 unter Verwendung einer Sonde vorliegen, bei welcher die Sondenspitze 1 mit den erfindungsgemäßen Einkerbungen 2 versehen ist. Durch die seitliche Anordnung dieser Einkerbungen 2 kann jetzt mit einer entsprechenden Führung des distalen Endes des Ureteroskops 9 durch den unteren Harnleiterabschnitt 6 hindurch ein seitwärts ausgerichtetes Fixieren des Steines 3 gegen die angrenzende Harnleiterwand erhalten werden, die auch für die jetzt über diese Einkerbungen eingeleitete Zertrümmerung des Steines beibehalten wird. Weil dabei gleichzeitig ein leichter Druck mit der Sondenspitze gegen den Stein ausgeübt wird und durch diesen Druck eine Außenwölbung 16 der Harnleiterwand erhalten wird, wird damit andererseits auch das Sichtfeld der Optik 14 des Ureteroskops 9 erweitert, sodaß neben der Verhinderung einer Steinpropulsion bei dem Arbeiten mit der erfindungsgemäßen Sonde auch die eigentliche Steinzertrümmerung unter entsprechend verbesserten Sichtverhältnissen durchgeführt werden kann. Diese Sichtverhältnisse können im übrigen noch mit der Maßnahme optimiert werden, daß die Sondenspitze mit Ausnahme der Einkerbungen dunkel bzw. lichtabsorbierend und die Einkerbungen hell bzw. lichtreflektierend ausgebildet werden, sodaß unmittelbar an der Stelle, wo die Steinzertrümmerung einsetzt, dann die besten Lichtverhältnisse vorliegen. Durch die seitliche Anordnung der Einkerbungen ist im übrigen gesichert, daß die Sondenspitze noch eine genügend große volle Mantelfläche aufweist, um eine Verletzung des Bereichs der Harnleiterwand zu verhindern, an welchem der Stein unfixiert bleibt.

## Patentansprüche

1. Metallsonde (10), die zur Verwendung bei der intrakorporalen Lithotripsie in das Lumen eines Endoskops (9) einführbar ist und deren distales Ende der Sonde (10) bildende Sondenspitze (1) für eine Zertrümmerung von Körpersteinen (3) durch eine pulsierende Translationsbewegung der Sondenspitze (1) genutzt wird, **dadurch gekennzeichnet, dass** die Sonde (10) ein Vollkörper mit konstantem kreisrunden Querschnitt und mit einer abgerundeten Sondenspitze (1) ist und die Sondenspitze (1) nahe dem Sondenende mit wenigsten einer seitlichen Einkerbung (2) versehen ist, die für ein seitwärts ausgerichtetes Fixieren des zu zertrümmernden Körpersteines (3) gegen eine angrenzende Ductuswand und zu dessen Zertrümmerung ausgebildet ist.

2. Metallsonde nach Anspruch 1 **dadurch gekennzeichnet, dass** die oder jede Einkerbung (2) als ein Zylindersegment ausgebildet ist.

3. Metallsonde nach Anspruch 1 oder 2, **dadurch gekennzeichnet, dass** die oder jede Einkerbung (2) als Quadersegment ausgebildet ist.

4. Metallsonde (10) nach einem der Ansprüche 1 bis 3, **dadurch gekennzeichnet, dass** die oder jede Einkerbung (2) als ein Vieleck ausgebildet ist.

5. Metallsonde (10) nach einem der Ansprüche 1 bis 4 **dadurch gekennzeichnet, dass** die oder jede Einkerbung (2) sägezahnförmig ausgebildet ist.

6. Metallsonde (10) nach einem der Ansprüche 1 bis 5, **dadurch gekennzeichnet, dass** bei mehreren Einkerbungen (2) alle Einkerbungen bezüglich der Längsachse der Sonde (10) gleich ausgerichtet sind.

7. Metallsonde (10) nach Anspruch 6, **dadurch gekennzeichnet, dass** die Einkerbungen (2) längs einer zu der Längsachse der Sonde (10)parallelen Mantellinie fluchtend ausgebildet sind.

8. Metallsonde (10) nach Anspruch 6 oder 7, **dadurch gekennzeichnet, dass** die Einkerbungen (2) mit Abstand zueinander angeordnet sind und für die Zwischenräume zwischen den Einkerbungen (2) der Nenndurchmesser der Sondenspitze (1) beibehalten wird.

9. Metallsonde (10) nach einem der Ansprüche 1 bis 8, **dadurch gekennzeichnet, dass** die Sondenspitze (1) mit Ausnahme der einen oder mehreren Einkerbungen (2) dunkel bzw. lichtabsorbierend und die Einkerbungen (2) hell bzw. lichtreflektierend ausgeführt sind.

## Claims

1. Metal probe (10) which, for use in intracorporal lithotripsy, can be inserted into the lumen of an endoscope (9), and whose probe tip (1) forming the distal end of the probe (10) is used for the destruction of stones (3) in the body through a pulsating translational movement of the probe tip (1),
**characterised in that**
the probe (10) is a solid body with a constant, circular cross-section and with a rounded-off probe tip (1), and close to the end of the probe the probe tip (1) is equipped with at least one lateral notch (2) which is designed for fixing the body stone (3) that is to be destroyed against an adjacent ductal wall, in a sideways direction, and for destroying it.

2. Metal probe in accordance with claim 1, **characterised in that** the - or each - notch (2) is designed as a cylinder segment.

3. Metal probe in accordance with claim 1 or 2, **characterised in that** the - or each - notch (2) is designed as a cuboid segment.

4. Metal probe (10) in accordance with one of the claims 1 to 3, **characterised in that** the - or each - notch (2) is designed as a polygon.

5. Metal probe (10) in accordance with one of the claims 1 to 4, **characterised in that** the - or each - notch (2) is designed in a saw-toothed shape.

6. Metal probe (10) in accordance with one of the claims 1 to 5, **characterised in that** in the case of several notches (2), all notches are aligned in the same way in respect of the longitudinal axis of the probe (10).

7. Metal probe (10) in accordance with claim 6, **characterised in that** the notches (2) are formed in alignment along a surface line that is parallel to the longitudinal axis of the probe (10).

8. Metal probe (10) in accordance with claim 6 or 7, **characterised in that** the notches (2) are arranged spaced apart from one another, and for the interspaces between the notches (2), the nominal diameter of the probe tip (1) is maintained.

9. Metal probe (10) in accordance with one of the claims 1 to 8, **characterised in that** the probe tip (1) - with the exception of the one notch or several notches (2) - is dark or light-absorbent, and the notches (2) are designed to be light or light-reflecting.

## Revendications

1. Sonde métallique (10) pouvant être introduite dans le passage d'un endoscope (9) lors d'une utilisation en lithotritie intra-corporelle, dont la pointe (1) formant l'extrémité distale de la sonde (10) est utilisée pour détruire des calculs (3) au moyen d'un mouvement de translation pulsé de la pointe de la sonde (1), **caractérisée en ce que** la sonde (10) présente un corps plein de section circulaire constante et une pointe (1) arrondie, et **en ce que** la pointe (1) de la sonde est équipée, près de l'extrémité de la sonde, d'au moins une encoche latérale (2) pour fixer latéralement le calcul (3) à détruire contre une paroi limitrophe et le détruire.

2. Sonde métallique selon la revendication 1, **caractérisée en ce que** la ou chaque encoche (2) est réalisée sous la forme d'un segment cylindrique.

3. Sonde métallique selon les revendications 1 ou 2, **caractérisée en ce que** la ou chaque encoche (2) est réalisée sous la forme d'un segment parallélépipédique.

4. Sonde métallique (10) selon une des revendications 1 à 3, **caractérisée en ce que** la ou chaque encoche (2) est de forme polygonale.

5. Sonde métallique (10) selon une des revendications 1 à 4, **caractérisée en ce que** la ou chaque encoche (2) est en dent de scie.

6. Sonde métallique (la) selon une des revendications 1 à 5, **caractérisée en ce que**, dans le cas de plusieurs encoches (2), toutes les encoches sont dirigées de la même manière par rapport à l'axe longitudinal de la sonde (10).

7. Sonde métallique (10) selon la revendication 6, **caractérisée en ce que** les encoches (2) sont alignées le long d'une génératrice parallèle à l'axe longitudinal de la sonde (10).

8. Sonde métallique (10) selon la revendication 6 ou 7, **caractérisée en ce que** les encoches (2) sont disposées à une certaine distance les unes des autres et le diamètre nominal de la pointe (1) de la sonde est conservé dans les espaces situés entre les encoches (2).

9. Sonde métallique (10) selon une des revendications 1 à 8, **caractérisée en ce que** la pointe (1) de la sonde, à l'exception de la ou des encoches (2), est de couleur sombre, respectivement absorbe la lumière, et les encoches (2) sont de couleur claire, respectivement réfléchissent la lumière.
